# EUROPEAN PATENT SPECIFICATION

(11) **EP 1 605 987 B1**
(45) Date of publication and mention of the grant of the patent: **20.02.2008**
(21) Application number: 04720466.4
(22) Date of filing: 12.03.2004
(51) Int. Cl.: A61M 25/00, A61B 18/18

(54) **OPTICALLY GUIDED PENETRATION CATHETERS AND THEIR METHODS OF USE**
OPTISCH GEFÜHRTE PENETRATIONSKATHETER UND IHRE ANWENDUNGSVERFAHREN
CATHETERS DE PENETRATION A GUIDAGE OPTIQUE ET LEUR MODE D'UTILISATION

(30) Priority: 13.03.2003 US 455015 P
(43) Date of publication of application: 21.12.2005
(73) Proprietor: Medtronic Vascular, Inc., Santa Rosa, CA 95403 (US)
(72) Inventor: MACAULAY, Patrick E., Windsor, CA 95492 (US); CHANG, John, Y., Mountain View, CA 94040 (US); KIM, Isaac, J., San Jose, CA 95135 (US); VRANY, Julia, D., Sunnyvale, CA 94086 (US)
(74) Representative: Coates, Ian Harold
(86) International application number: PCT/US2004/007720
(87) International publication number: WO 2004/082738

(56) References cited:
- WO-A-99/48545
- WO-A-99/49780
- WO-A-02/056937
- US-A- 6 030 377
- US-A- 6 071 292
- US-A- 6 117 128
- US-A1- 2001 027 316
- US-B1- 6 456 874

## Description

### FIELD OF THE INVENTION

The present invention related generally to medical devices and treatment methods, and more particularly to optically guided catheters that can be used to penetrate from one location within the body to another location, and methods for using such catheters for diagnostic and/or therapeutic purposes.

### BACKGROUND OF THE INVENTION

There exist numerous situations in which it is desirable to utilize penetration catheter(s) to penetrate from the lumen of an anatomical conduit (e.g., a blood vessel, urethra, esophagus, trachea, bronchus, fallopian tube, etc.) to a location outside of that lumen (e.g., a location within the wall of the anatomical conduit or a location beyond the wall of the anatomical conduit). Examples of such procedures are described in various prior patents including but not limited to Patents Nos. 5,830,222, 6,068,638 and 6,071,292, copending United States Patent Application Nos. 08/730,327, 09/056,589, 09/282,276 and 09/282,774 and certain non-patent publications such as, Osterle, Stephen N. et al., Percutaneous In Situ Coronary Venous Arterialization: Report of the First Human Catheter-Based Coronary Artery Bypass, Circulation, 2001;103:2539-2453.

The prior art has included certain penetration catheters that may be used to carry out the above-mentioned procedures or to otherwise penetrate from the lumen of an anatomical conduit in which the catheter is positioned to a location outside of that lumen (e.g., a location within the wall of the anatomical conduit or a location beyond the wall of the anatomical conduit). These penetration catheters typically have penetrators, such as sharp-tipped needles, that advance from the catheter and into or through the wall of the anatomical conduit into which the catheter is positioned. Some of these penetration catheters are equipped with catheter orientation and/or guidance apparatus to allow the operator to pre-orient or specifically position the catheter within the anatomical conduit or to otherwise project the trajectory on which the penetrator will advance such that, when the penetrator is subsequently advanced from the catheter, the penetrator will enter a discrete target location that is within or beyond the wall of the anatomical conduit. Examples of such guided penetration catheters are described in United States Patent Nos. 5,830,222, 6,068,638 and 6,071,292 or copending United States Patent Application Nos. 08/730,327, 09/056,589, 09/282,276 and 09/282,774. At least some of the previously described guided penetration catheters utilize imaging (e.g., radiographic imaging, ultrasound, radiofrequency mapping, etc) to facilitate the pre-orientation or specific positioning of the catheter prior to advancement of the penetrator. Recent advancements in imaging technologies have included certain new imaging techniques that may be useable in connection with these guided penetration catheters.

PCT International Patent Publication WO-A-02/056937 describes methods and apparatus for delivery of substances or apparatus to target sites located outside blood vessels within the body of a human or animal patient. A vessel wall penetrating catheter is inserted into the vasculature, positioned and oriented within a blood vessel near the target extravascular site and a penetrator is advanced from the catheter so as to penetrate outwardly through the wall of the blood vessel in the direction of the target site. Thereafter, a delivery catheter is passed through a lumen of the penetrator to the target site. A desired substance or apparatus is then delivered to or obtained from the target site. In some applications, the penetrator may be retracted into the vessel wall penetrating catheter and the vessel wall penetrating catheter may be removed, leaving the delivery catheter in place for chronic or continuous delivery of substance(s) to and/or obtaining of information or samples from the target site. Alternatively, a delivery catheter having an occlusion member or balloon may be advanced into a vein or venule and the occlusion member or balloon may be used to occlude the lumen of the vein or venule during and after injection of a substance through the catheter, such that the substance will not be carried away by normal venous blood flow and will remain in the vein or venule for a sufficient period of time to have its intended effect (e.g. to enter adjacent tissues through capillary beds drained by that vein or venule). WO-A-02/056937 does not describe the placement of any OCT device within the delivery catheter or the use of OCT to distinguish target tissue from surrounding tissue based on some parameter of the tissue or to guide the advancement of the delivery catheter to a particular target location.

One area in which considerable development is occurring is in the field of optical imaging. The present invention generally comprises penetration catheter of the above-described nature which incorporate or utilize optical imaging to guide the positioning of the catheter within the anatomical conduit lumen and/or to guide advancement of the penetrator to a specific target location within or beyond the wall of the anatomical conduit in which the catheter is positioned.

One specific optical imaging technique which may be used in this invention is known as Optical Coherence Tomography (OCT). OCT infrared light waves are reflected off of the internal microstructures of biological tissues. The frequencies and bandwidths of the infrared light used in OCT are substantially higher than medical ultrasound signals, thus resulting in substantially better image resolution than with ultrasound imaging. Infrared light may be delivered to the imaging site through an optical fiber or other suitable waveguide. In this invention, the optical fiber or other light transmitting waveguide may be integrated into the penetration catheter or may be inserted into a lumen within the penetration catheter or may be advanced into the body separately from the penetration catheter. In this regard, OCT may be used to visualize a target location (e.g., the lumen of another anatomical conduit, a tumor or tissue mass, a location defined by anatomical landmarks, etc.) and to facilitate advancement of the catheter's penetrator into that target location. Additionally, OCT may be used to dynamically visualize and quantify metabolic, physiologic, genetic and/or developmental changes in tissue that cannot be assessed by traditional medical imaging methods. In this regard, OCT may be used to locate a specific target location which is distinguishable from surrounding anatomical structures or tissue due to the presence or absence of some specific metabolic, physiologic, genetic and/or developmental changes and to guide the catheter's penetrator into that target location. Alternatively to OTC, this may be accomplished by other technologies such as infrared imaging, ultrasound, local magnetic resonance imaging, other magnetic imaging, radiofrequency detector, electrode(s) (e.g., pH, pO2, pCO2, evoked potential, galvanic response, tissue movement sensor (e.g:, accelerometer, strain gage), sensors for specific drugs or substances (e.g., neurotransmitter levels, insulin levels, dopamine levels, etc).

OTC and/or the other above-mentioned alternative imaging/sensing technologies are useable to sense and locate tissue having a specific characterization parameter (e.g., a metabolic rate; pH; gas content; temperature; motion; contractility; ischemia; fluid content; perfusion; physiologic, genetic and/or developmental change; infarct; necrotic area; viability; substance content; etc.). This ability to target specific locations on the basis of such tissue characterization parameters renders the present invention useable for such applications as; targeting a specific lesion within tissue or the vasculature, targeting vulnerable plaque, distinguishing infarcted or necrotic tissue from viable tissue, locating specific neurons or nuclei within the CNS, distinguishing between ischemic and non-ischemic tissue, targeting tumor or neoplastic lesions,

In OTC, an interrogating beam of light is cast into tissue and a technique known as interferometry is utilized to determine the path length traveled by the interrogating beam incident on the tissue. This is commonly accomplished by dividing the source light into two beams with an apparatus known as an interferometer, and then directing one of the beams into the tissue and the other beam to a reference mirror positioned at a known location relative to the beam source. The light which returns from both the tissue and the reference mirror enters a detector where it is recombined and the interference between the two beams is determined. A property of light known as its "coherence length" determines the axial or depth resolution of the OCT system. Thus, light having a specific coherence length may be selected for use in a particular application based on the specific nature of the tissue through which the penetrator must travel.

One interferometer design used in OCT is known as a Michelson Interferometer. In a Michelson Inferometer, a reflective beam splitter is used to split the beam of light and channel a portion of the light through a reference arm and the other portion of the light through a sample arm. Light reflected from each arm is recombined in this same element. By scanning the location of the reference mirror in the interferometer, the magnitude of the light reflected back from the sample is determinable as a function of depth. This is known referred to as an "axial scan." To acquire a two-dimensional image, axial scans are performed rapidly while the sample beam is moved across the tissue. One OCT imaging catheter that may be suitable for use in this invention is a 2.0 mm diameter catheter through which there extends a single mode optical fiber within a wound stainless steel cable. At the distal tip of the fiber, a gradient index lens and micro-prism may be positioned to produce a focused output beam that propagates transversely to the catheter axis. At the proximal end of the OCT catheter, the cable may be fastened to a sliding carriage. The carriage is then translated linearly (e.g., by computer control) to carry out transverse scanning of the interrogating beam. As this carriage translates, the cable and optical components slide within the sheath so that the interrogating beam is swept longitudinally along the catheter axis. Further details of the construction and operation of inferometers used for OCT applications are published and readily available and/or are known to those of skill in the art of designing optical imaging systems of this type. One specific area where OCT imaging may be used in the present invention is to control not only the trajectory of the penetrator but also the depth to which the penetrator is advanced to ensure that the tip of the penetrator is positioned within the desired target location. This is particularly useful in situations where it is desired to position the tip of the penetrator in a specific tissue region or layer (e.g., a specific layer of a blood vessel wall, within the parenchyma of a tumor, within a certain tissue type, etc.) In this regard, OCT has proven to be useable to image and discern the relative thickness of different layers of tissue in the wall of an anatomical conduit (e.g., the intima, muscularis, adventitia of an artery or the epithelium, lamina propria, muscularis mucosa, submucosa and muscularis propria of the esophagus). Thus, OCT can be used to guide the positioning of a catheter's penetrator within not only a specific anatomical structure but also within a specific tissue layer of that anatomical structure.

### SUMMARY OF THE INVENTION

In general, an optically guided penetrating catheter system of the present invention comprises an elongate a) a catheter body that is positionable within the lumen of an anatomical conduit, b) a tissue penetrator that is advanceable from the catheter body to a target location within or outside of the wall of the anatomical conduit in which the catheter body is positioned and c) an optical imaging/penetrator guidance element useable to obtain an optical image (e.g., an OCT image) of the target location relative to at least one of i) the trajectory upon which the penetrator will subsequently advance from the catheter body and/or ii) the distance between the catheter body and the target location.

In accordance with the invention, in instances where the optical image of the target location is related to the trajectory on which the penetrator will subsequently advance from the catheter body, such image will enable the operator to adjust the position and/or rotational orientation of the catheter body within the lumen of the anatomical conduit to whatever extent is necessary to ensure that when the penetrator is subsequently advanced from the catheter body, the tip of the penetrator will enter the desired target location within or outside of the wall of the anatomical conduit. In such instances, the optical imaging apparatus may interact with a marker or other indicator of the projected penetrator trajectory such that the displayed image will show the target location along with an indicator (e.g., a line, vector, pointer, echo, artifact, electronic marking, light, etc.) of the projected penetrator trajectory. The operator may then move and/or rotate the catheter within the lumen of the anatomical conduit until the indicator of the projected penetrator trajectory coincides with the image of the target location indicating that subsequent advancement of the penetrator will cause the penetrator to enter the target location.

Further in accordance with the invention, in some instances, the penetration catheter may be inserted into a man made lumen or passageway, such as a neolumen created within the wall of a blood vessel and the optical imaging apparatus may be used to locate the true lumen of the blood vessel and to guide advancement of the penetrator into such true lumen. Examples of such procedures are described in copending United States Patent Application No. 09/860,147 entitled Methods for Bypassing Total or Near-Total Obstructions in Arteries or Other Anatomical Conduits. As used herein the therm "lumen" includes any passageway or cavity within the body, whether natural or man-made.

Still further in accordance with the invention, the relationship between the image of the target location and the In this manner, the optical image obtained from the optical imaging element may be used by the operator to adjust the position and/or rotational orientation of the catheter body within the anatomical conduit lumen and/or the depth to which the penetrator is advanced, such that the penetrator will advance into the target location. The optical image of the target location may be related to the trajectory on which the orientation element may comprise imageable marking(s), sensor(s), imaging transducer(s), electro-anatomical mapping and catheter guidance system(s) or any other suitable type of apparatus or system useable to predict the direction or track on which the penetrator will advance from the catheter body, including but not necessarily limited to those described in United States Patent Nos. 5,830,222, 6,068,638 and 6,071,292 and published PCT International Patent Application Nos. PCT/US99/07115, PCT/US99/07112 and unpublished United States-designating PCT International Patent Application No. PCT/US02/03941.

Further aspects and elements of the present invention may be set forth in the following detailed description and the accompanying drawings to which it refers.

### BRIEF DESCRIPTION OF THE DRAWINGS

Figure 1 is a schematic diagram of a human patient into whom a catheter system of the present invention has been inserted.
Figure 2 is a cross sectional image through a vein in which an optically guided penetration catheter of the present invention is positioned and an adjacent artery, wherein the catheter is properly rotated such that a penetrating member will advance from the catheter through the wall of the vein and into the artery.
Figure 3 is a cross sectional image through a vein in which an optically guided penetration catheter of the present invention is positioned and an adjacent artery, wherein the catheter is rotated such that a penetrating member will advance from the catheter through the wall of the vein to some location outside the artery.
Figure 4 is a side elevational view of the handpiece portion of one embodiment of a catheter device of the present invention, wherein the handpiece is configured such that the penetrator of the catheter is in its retracted position.
Figure 5 is a longitudinal sectional view of the distal portion of one embodiment of a catheter device of the present invention, wherein the penetrator is in its retracted position.
Figure 5A is a cross sectional view through line 5A-5A of Figure 5.
Figure 6 is a side elevational view of the handpiece portion of one embodiment of a catheter device of the present invention, wherein the handpiece is configured such that the penetrator of the catheter is in its extended position.
Figure 7 is a longitudinal sectional view of the distal portion of one embodiment of a catheter device of the present invention, wherein the penetrator is in its extended position.
Figure 8 is a longitudinal sectional view through the distal portion of another embodiment of a catheter device of the present invention.
Figure 8A is a cross sectional view through line 8A-8A of Figure 8.
Figure 8B is a schematic diagram illustrating an example of an OCT image obtained from a catheter device of the present invention that is positioned within an artery adjacent to a vein.
Figure 9 is a partial cut-away view of a microcatheter having an OCT apparatus positioned therein being advance through the lumen of a needle to a target location within a patient's body.

### DETAILED DESCRIPTION

The following detailed description and the accompanying drawings are provided for the purpose of describing and showing certain embodiments or examples of the invention only. No attempt has been made to exhaustively describe all possible embodiments and examples of the invention. Thus, this detailed description and the accompanying drawings do not limit the scope of the invention in any way.

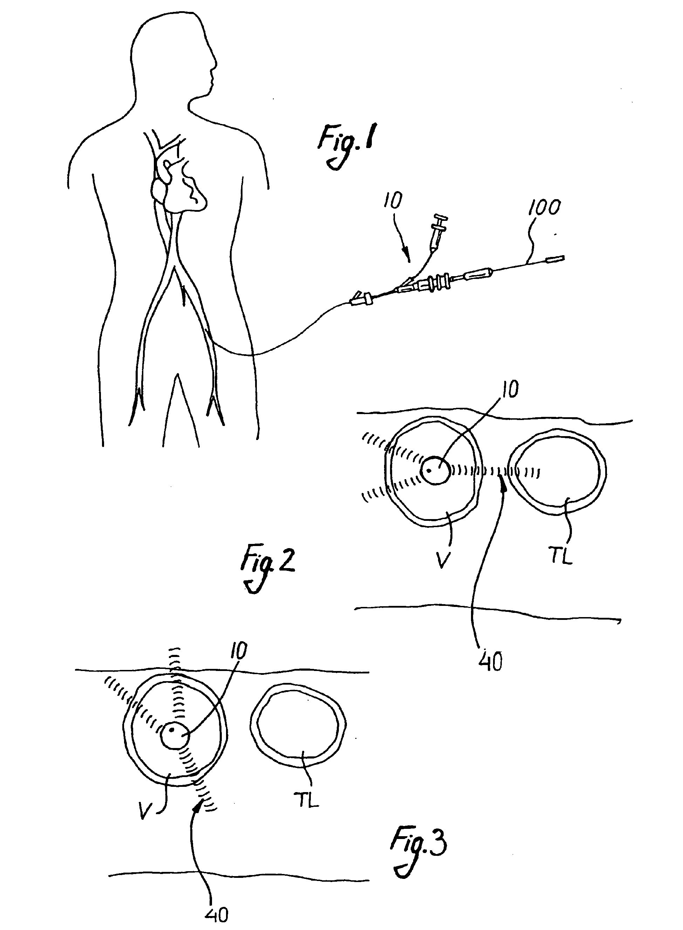

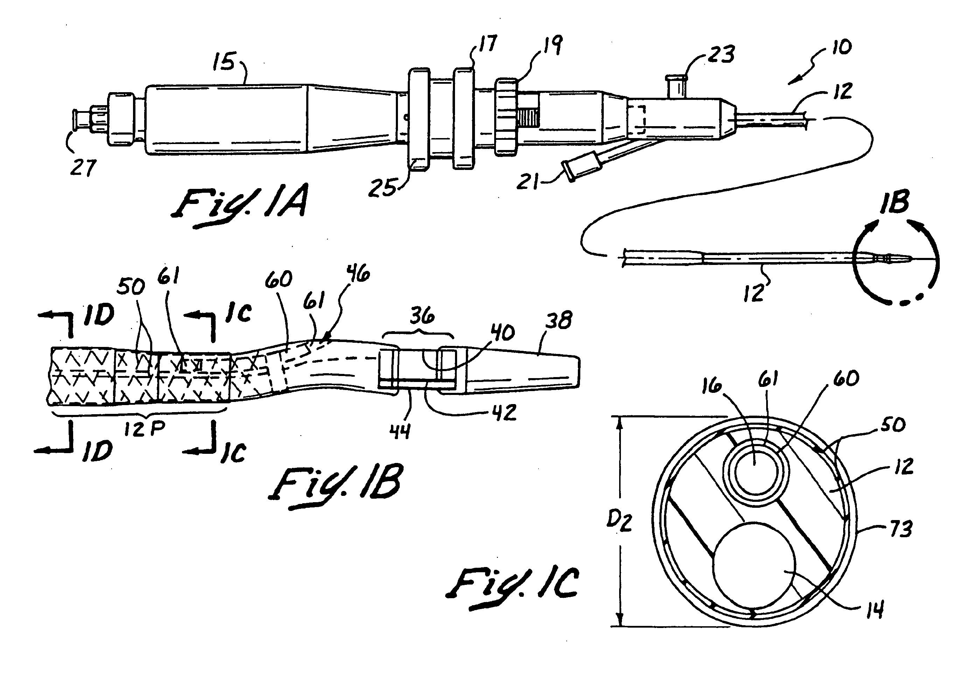

Figures 1-7 show several different embodiments of optically guided penetrating catheters of the present invention and examples of some of the methods by which such catheters may be used.

### A. A First Embodiment:

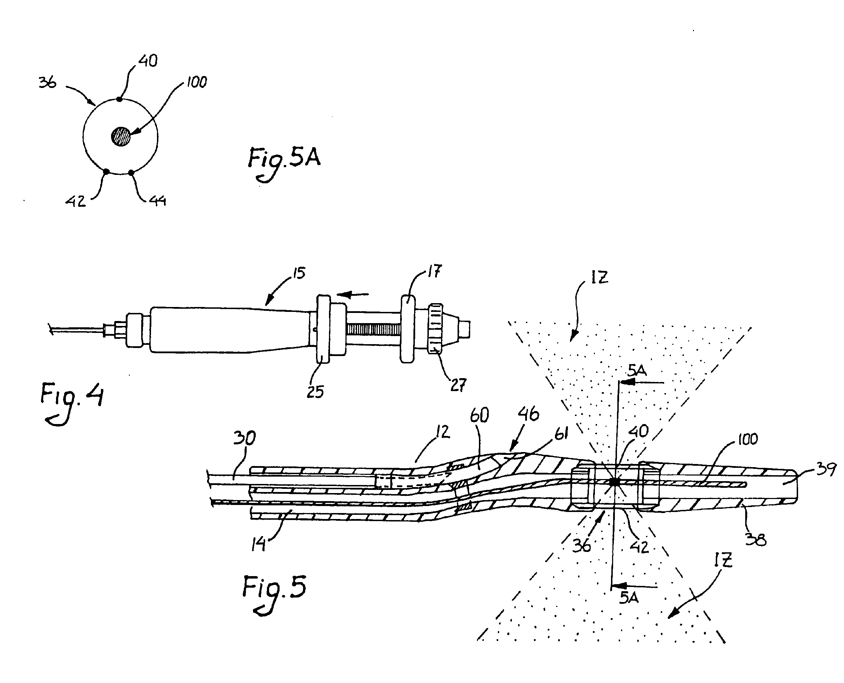

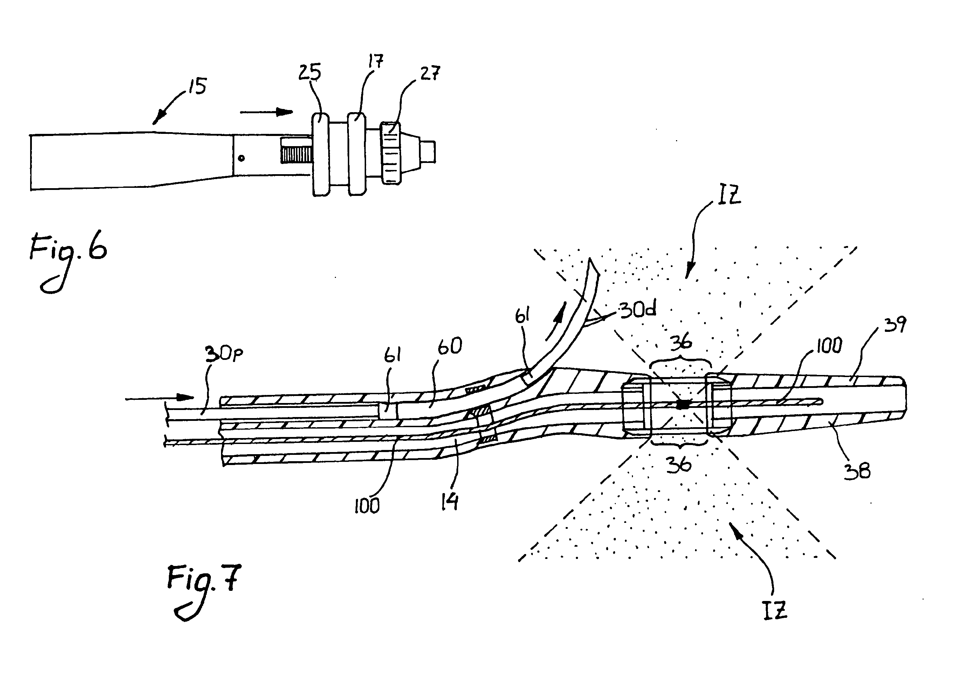

Referring to Figures 1-7 there is shown a first embodiment of a tissue-penetrating catheter device 10 which is insertable into the lumen of a blood vessel or other anatomical conduit and useable to penetrate from the lumen of the anatomical conduit to a specific target location situated outside the lumen of the anatomical conduit (i.e., within the wall of the anatomical conduit or outside the wall of the anatomical conduit. This catheter device 10 generally comprises an elongate, flexible catheter body 12 having a proximal portion 12_{P} of a first diameter D₁ and a distal portion 12_{D} of a second diameter D₂ which is smaller than the first diameter D₁. In this embodiment, the catheter body 12 has at least two (2) lumens 14, 16 which extend longitudinally therethrough. The first lumen 14 is sized and configured to permit an optical imaging apparatus, such as an OTC core or wire (e.g., OCT system and OCT probes manufactured by Lightlab Imaging, Inc., Westford, MA) to be inserted therethrough and slidably disposed therewithin. The second lumen 16 is sized and configured to house a tissue-penetrating member 30. This tissue-penetrating member may comprise a solid or hollow needle. The penetrating member 30 is alternately moveable between i) a retracted position (Figure 4-5) wherein the distal end DE of the needle member 30 is contained within the catheter body 12, and ii) an extended position (Figures 6-7) wherein the needle member 30 is advanced out of the catheter body 12 so as to penetrate into or through the wall of the blood vessel or other anatomical conduit in which the catheter body 12 is positioned.

An orientation structure 36 and tip member 38 are formed integrally with or mounted on the distal end of the catheter body 12, as may be appreciated from Figures 1b, 5, 5a and 7. The orientation cage 36 comprises first 40, second 42 and third 44 strut members which extend longitudinally between the distal end of the catheter body 12 and the proximal end of the distal tip member 38. The first strut member 40 is in known alignment with the trajectory on which the penetrating member 30 will advance from the catheter body 12. The second and third strut members 42, 44 are located at equally spaced distances from the first strut member 40, while the distance between the second and third strut members 42,44 is less than the distance between either of those second and third strut members 42, 44 and the first strut member 40. Such disparate (e.g., unequal) radial spacing of these strut members 40, 44 and 46 allows the operator to easily identify and distinguish the artifact created by the first strut member 40 from the artifacts created by the other two strut members 42, 44 on an image received from the optical imaging apparatus 100 positioned within the orientation structure 36. Thus, in this manner, the operator may selectively rotate the catheter body 12 until the bloom or artifact emanating from the first strut member 40 is directly aligned with or is pointing to the target location into which the penetrating member 30 is to be advanced. An illustration of this technique is shown in Figures 2 and 3. Figure 2 shows the optical image which is obtained when the tissue-penetrating catheter 10 is properly rotated such that the artifact 40(artifact) of the first strut member 40 is aligned with the target location and the penetrating member 30 will advance into such target location. Figure 3 shows another situation where the tissue-penetrating catheter 10 is not properly rotated, the artifact 40(artifact) created by the first strut member 40 is not aligned with the target location and the needle member 30, if advanced at that time, would not enter the target location.

It will be appreciated that the disparate distancing of the strut members 40, 42, 44 is only one of numerous possible ways of rendering the first strut member 40 distinguishable from the other two strut members 42, 44. Alternatively, the size or configuration of the first strut member could be different so as to produce a distinguishable ultrasound image or the material or surface characteristics of the first strut member 40 could be made different from the other two strut members 42, 44 such that the first strut member 40 would reflect more or less ultrasound than the other two strut members 42, 44 thus producing an ultrasound image which is distinguishable from the images produced by the other two strut members 42, 44. It will also be appreciated that only one strut member may be required to provide a distinguishable element to aid catheter orientation, or alternatively two strut members may be positioned to delineate a zone within which the tissue-penetrating member may be deployed, or other procedure conducted.

The distal tip member 38 is preferably of blunt tipped configuration and is formed of smooth soft material (e.g., PEBAX having a durometer hardness of 35D) so as to minimize trauma to the vasculature as the tissue-penetrating catheter device 10 is advanced or otherwise manipulated about. A hollow lumen 39 may extend longitudinally through the tip member 38, in alignment with the first lumen 14 of the catheter body 12, such that an OCT catheter, OCT wire or other optical imaging device and/or a guidewire or other apparatus may be advanced from the first lumen 14, through the orientation structure 36, through the distal tip lumen 38 and distally beyond the catheter device 10. Such advancement of an optical imaging device (e.g. OCT catheter or OCT wire) beyond the distal end of the catheter 10 may allow the operator to use the optical imaging apparatus to explore areas which are ahead of the distal end of the tissue-penetrating catheter without having to advance the tissue-penetrating catheter from its then-present position. The ability to pass a guidewire through lumen 14 also allows the optically guided penetrating catheter 10 to be introduced into a body lumen in a traditional "over the wire" manner.

The tissue penetrating element may comprise and suitable tissue penetration means suitable for the intended application, including but not limited to penetrating members 30 such as solid or hollow needles, tissue penetrating apparatus such as electrosurgical devices, blade(s), rotating cutters, etc. or flows of tissue-penetrating energy such as laser beams capable of creating the desire penetration tract through tissue. In the particular embodiment shown in Figures 1-7, there is provided a tissue penetrating member 30 which comprises a hollow needle having a proximal shaft 30p formed of stainless steel hypotubing and a resilient, curved distal portion 30d formed of a resilient material or, more preferably, a material such as NiTi alloy. Preferably a lumen 31 extends longitudinally through the proximal shaft 30p and the curved distal portion 30d.

The particular radius of curvature of the curved distal portion 30d may be an important factor in determining the trajectory and path of the needle tip as it advances and the point at which the needle tip will stop when in its fully advanced position.

The distal tip of the needle member 30 is preferably sharpened so as to easily penetrate through the walls of the blood vessels and any intervening tissue located therebetween. One preferred needle tip configuration is a lancet-type bevel which provides excellent tissue-penetrability and retains its sharpness after multiple retractions into/advancements from the catheter.

In many applications, the controllability and aiming of the needle member 30 may be enhanced by constraining the needle member 30 such that it will remain in a preferred plane or acceptable penetration zone relative to the target location. In embodiments where a curved needle member 30 is advanced out of a side aperture of the catheter 10, any rotation of the needle member 30 prior to, during or after its advancement can cause the distal end of the curved needle member 30 to deviate from or move out of the intended plane or acceptable penetration zone. In this regard, the potential for such unwanted lateral movement of the distal end of the needle member 30 may be prevented or substantially limited by providing a stabilizer to prevent or substantially limit the amount of rotation that the needle member 30 may undergo relative to the catheter body 12 or to otherwise prevent or deter the needle member from deviating from a predetermined acceptable penetration zone as it is advanced from the catheter 10. Such prevention or limitation of the potential for rotation or lateral movement of the needle member 30 may be accomplished in any suitable way. One particular way to stabilize such needle member 30 is by way of a needle housing assembly as shown in figures 1b, 1c, 5 and 7. This assembly comprises a curved needle housing 60 which mates with the preformed curvature of the needle member 30 to deter rotation of the needle member 30. Such needle housing 60 comprises a curved, rigid tube. A tubular liner 61 may be disposed within, and may extend from either end of, the curved needle housing 60. Such tubular liner 61 may be formed of a three-layer composite wherein the inner layer is a lubricious polymer material (e.g., polytetrafluoroethylene (PTFE)), the middle layer is a structural polymer material (e.g., polyimide) and the outer layer is an adhesive material which will bond to the inner surface of the curved needle housing 60 and to the inner surface of the needle lumen 16 at either end of the housing 60 (e.g., polyurethane adhesive). When the needle member 30 is in its retracted position, and during advancement, the portion of the needle member which resides within the needle housing 60 will remain in a slightly curved state in conformance to the slightly curved configuration of the needle housing 60. This serves to deter the needle member 30 from rotating relative to the catheter body 12 and/or from undergoing uncontrolled movement out of the intended acceptable penetration zone during or after advancement from the catheter. This prevention or deterrence from rotation of the needle member 60 allows the operator to control the orientation of the lancet type or other bevel formed in the needle tip, and also enhances the operator's ability to predict the precise position of the needle tip by eliminating or minimizing the uncontrolled side-to-side movement of the needle. The details of such penetrator stabilization and the manner in which the needle stabilizing assembly may be manufactured and mounted within the catheter 10 is described in copending United States Patent Application SN 09/282,276 which is currently in the allowance process and corresponding PCT International Patent Publication No. WO99/49793.

The catheter body 12 may include a relatively stiff proximal section, a less stiff medial section, and a distal section shown in Figs 1A and 1B. In this manner, the catheter body 12 may exhibit varying flexibility and torque strength along its length, and may incorporate reinforcement members such as a reinforcement braid member which imparts structural integrity as well as enhancing the ability of the catheter body to transmit torque. A hand piece 15 is attached to the proximal end of the catheter body 12, as shown. In an embodiment suitable for adult cardiac applications the hand piece 15 and proximal section of the catheter body 12 may be approximately 115cm in length, the medial section of the catheter body 12 may extend approximately 25cm. The proximal and medial sections of the catheter body 12 may contain a braided component 50 as shown in Fig. 1B, encased in a polymer material (e.g. Pebax, nylon, polyurethane, polyester or PVC) extruded to form the inner lumen 50b and out jacket 50a of catheter body 12.

### B. Other Embodiments/Variations:

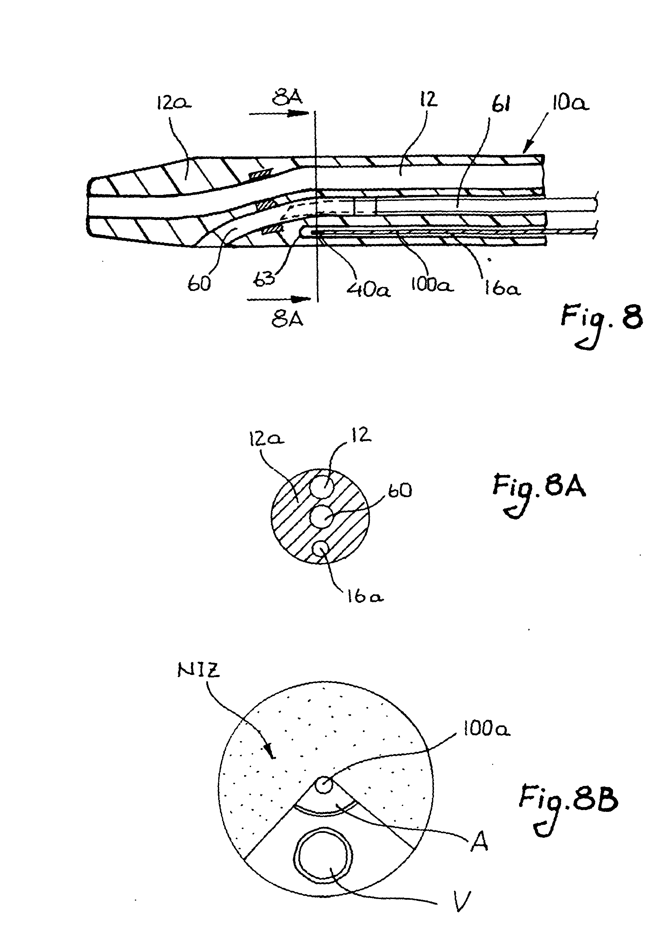

Figures 8-9 show various alternative embodiments or modifications of the above-described first embodiment of the optically guided penetration catheter 10.

In the alternative embodiment or modification shown in Figures 8-8b, the catheter10a has a construction similar to that of the above described first embodiment, except that the catheter 10a has at least three lumens, including a guidewire lumen 12, a penetrator lumen 61 and a optical imaging apparatus receiving lumen 16a which terminates distally in a closed or "blind" end 63 located within the catheter body adjacent to the needle housing 60, as shown in Figure 8 and in the cross sectional view of Figure 8a (taken through Line A-A of Figure 8).

In operation, the catheter is inserted into the lumen of a blood vessel or other anatomical conduit and the optical imaging apparatus 100 is mounted, inserted or otherwise positioned in the optical imaging apparatus receiving lumen 16a. The catheter body could be optically transparent or a lens or window may be formed in the catheter body to allow the energy to be transmitted from and received by the OTC apparatus positioned within lumen 16a.

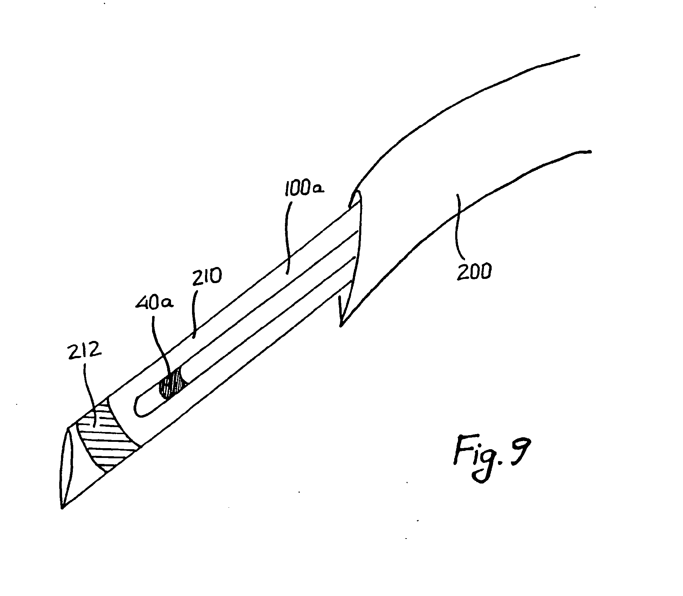

Figure 9 shows another embodiment wherein a needle 200 is inserted into the patient's body directly or advanced from a catheter (e.g. a catheter such as either of the embodiments shown above or any other type of catheter having a port located in the distal end or side wall of the catheter through which a needle or penetrator may be advanced). A second catheter 210 is advanced through the lumen of the needle 200 and an optical imaging apparatus 100 (e.g., an OCT wire or OCT core) is positioned within or inserted into the lumen of that second catheter 210 so as to obtain an image of the distal end of the second catheter as well and an image of the target location, thereby enabling the operator to guide the advancement of the second catheter 210 into the desired target location. A marker band 212 may be formed or mounted on the second catheter to provide a clear image or artifact on the image obtained form the optical imaging apparatus 100, thereby assisting the operator in visualizing the distal tip of the second catheter 210 as it advances toward the target location. Substances or devices may then be delivered through the second catheter in the manner described in copending United States Patent Application No. 09/766,502 entitled Methods and Apparatus for Acute or Chronic Delivery of Substances to Extravascular Treatment Sites and PCT International Patent Publication No. WO02/056937 Entitled Devices, Systems and Methods for Acute or Chronic Delivery of Substances or Apparatus to Extravascular Treatment Sites.

### C. Examples of Procedures Which Use The Optically Guided Penetration Catheters

As may be appreciated from the foregoing description, the penetration catheters 10, 10a may be used to perform or facilitate a broad range of diagnostic or therapeutic procedures in human or veterinary patients. For example, the penetration catheter 10, 10a may be positioned within the lumen of a blood vessel or within another anatomical structure and the penetrator 30, 60, 210 may then be advanced, under optical guidance, to a desired target location. The target location may be within or outside of the wall of the particular vessel, organ or other anatomical structure in which the penetration catheter is positioned. In some cases, as illustrated in Figures 2 and 3, the target location may be within the lumen of another blood vessel. In other cases, the target location may be a body cavity, organ, tumor, lesion, area of infracted or ischemic tissue, etc. In some cases where the penetrator comprises a hollow needle, a guidewire or other apparatus (e.g., an electrode, sensor, stimulator, transponder, transmitter, receiver, apparatus for delivering therapy, drug delivery apparatus, catheter, etc.) may then be advanced through the lumen of the penetrator 30, 60, 210 and into the target location. Also, in some cases where the penetrator comprises a hollow needle, a therapeutic or diagnostic substance (e.g., a medicament, drug, therapeutic agent, diagnostic agent, myocytes, myoblasts, other cells, genetic material, gene therapy preparation, protein, dye, radiopaque material, etc.) may be delivered through the lumen of the penetrator 30, 60, 210 and into the target location.

Although exemplary embodiments of the invention have been shown and described, many changes, modifications and substitutions may be made by those having ordinary skill in the art without necessarily departing from the scope of this invention. Specifically, elements or attributes described in connection with one embodiment may also be used in connection with another embodiment provided that the inclusion or use of such element or attribute would not render the other embodiment in which it is incorporated unusable or otherwise undesirable for the intended application. Accordingly, all such additions, deletions, modifications and variations to the above-described embodiments are to be included within the scope of the following claims.

## Claims

1. A penetration catheter system comprising:
an elongate catheter that is insertable into a natural or man-made lumen within the body of a mammalian patient;
a penetrator (200) having a penetrator lumen, said penetrator being advanceable from the catheter to a target location outside of the lumen in which the catheter is positioned; and
a second catheter (210) having a distal end and a lumen, said second catheter being advanceable through the lumen of the penetrator (200);
**characterized in that** the penetration catheter system further comprises an optical coherence tomography apparatus (100, 100a) positioned within or insertable into the lumen of the second catheter (210) and useable to guide advancement of the second catheter (210) to the desired target location.

2. A catheter system according to Claim 1 wherein the optical coherence tomography apparatus comprises an optical coherence tomography wire that is insertable into the lumen of the second catheter.

3. A catheter system according to Claim 1 wherein the optical coherence tomography apparatus comprises an optical coherence tomography core that is insertable into the lumen of the second catheter.

4. A catheter system according to any preceding claim wherein the catheter system further comprises at least one marker (212) formed or mounted on the second catheter to provide an image or artifact on the image obtained form the optical coherence tomography apparatus (100, 100a).

5. A catheter system according to Claim 4 wherein said at least one marker (212) comprises a marker which indicates the position of the distal end of the second catheter.

6. A catheter system according to Claim 5 wherein said at least one marker comprises a marker band located adjacent to the distal end of the second catheter.

7. A catheter system according to any preceding claim further in combination with apparatus for delivering a therapeutic or diagnostic substance through the second catheter.

8. A catheter system according to Claim 7 further in combination with a quantity of a therapeutic or diagnostic substance deliverable through the lumen of the second catheter.

9. A catheter system according to Claim 8 wherein the therapeutic or diagnostic substance is selected from the group of: medicaments, drugs, therapeutic agents, diagnostic agents, myocytes, myoblasts, other cells, genetic material, gene therapy preparations, proteins, dyes and radiopaque materials.

10. A catheter system according to any preceding claim wherein the optical coherence tomography apparatus is operative to distinguish the target location from surrounding anatomical structures or tissue based on the presence or absence of a metabolic, physiologic, genetic and/or developmental factor.

11. A catheter system according to any preceding claim wherein the penetrator comprises a hollow needle.

12. A catheter system according to any preceding claim wherein the optical coherence tomography apparatus is operative to locate the target location by sensing a characterization parameter selected from the group of: metabolic rate, pH, gas content, temperature, motion, contractility, ischemia, fluid content, perfusion, presence of infarction; necrosis, viability and content of a particular substance.

13. A catheter system according to any preceding claim wherein the optical coherence tomography apparatus is useable to determine the location of a target location selected from the group of: vulnerable plaque, infarcted tissue, necrotic tissue, viable tissue, specific neurons within the central nervous system, specific nuclei within the central nervous system, ischemic tissue, non-ischemic tissue, tumors and neoplastic lesions.

## Patentansprüche

1. Penetrations-Katheter-System mit:
einem langgestreckten Katheter, der in ein natürliches oder von Menschen hergestelltes Lumen innerhalb des Körpers eines Säugetier-Patienten einführbar ist;
einem Penetrator-Element (200), dass ein Penetrator-Lumen aufweist, wobei das Penetrator-Element von dem Katheter aus zu einem Zielort außerhalb des Lumens vorschiebbar ist, in dem der Katheter angeordnet ist; und
einem zweiten Katheter (210), der ein distales Ende und ein Lumen aufweist, wobei der zweite Katheter durch das Lumen des Penetrator-Elementes 8200) vorschiebbar ist;
**dadurch gekennzeichnet, dass** das Penetrations-Katheter-System weiterhin eine optische Kohärenztomographie-Vorrichtung (100, 100a) umfasst, die in dem Lumen des zweiten Katheters (210) angeordnet oder in dieses einführbar und zum Führen der Vorwärtsbewegung des zweiten Katheters (210) zum gewünschten Zielort verwendbar ist.

2. Katheter-System nach Anspruch 1, bei dem die optische Kohärenztomographie-Vorrichtung einen optischen Kohärenztomographie-Draht umfasst, der in das Lumen des zweiten Katheters einführbar ist.

3. Katheter-System nach Anspruch 1, bei dem die optische KohärenzTomographie-Vorrichtung einen optischen Kohärenztomographie-Kern umfasst, der in das Lumen des zweiten Katheters einführbar ist.

4. Katheter-System nach einem der vorhergehenden Ansprüche, bei dem das Katheter-System weiterhin zumindest ein Markierungs-Element (212) umfasst, das auf dem zweiten Katheter ausgebildet oder befestigt ist, um ein Bild oder ein Artifakt auf dem Bild zu liefern, das von der optischen Kohärenztamagraphie-Vorrichtung (100, 100a) gewonnen wird.

5. Katheter-System nach Anspruch 4, bei dem das zumindest eine Markierungs-Element (212) ein Markierungs-Element umfasst, das die Position des distalen Endes des zweiten Katheters anzeigt.

6. Katheter-System nach Anspruch 5, bei dem das zumindest eine Markierungs-Element ein Markierungs-Band umfasst, das benachbart zu dem distalen Ende des zweiten Katheters angeordnet ist.

7. Katheter-System nach einem der vorhergehen Ansprüchen, weiterhin in Kombination mit einer Vorrichtung zur Zuführung einer therapeutischen oder diagnostischen Substanz durch den zweiten Katheter.

8. Katheter-System nach Anspruch 7, weiterhin in Kombination mit einer Menge einer therapeutischen oder diagnostischen Substanz, die über das Lumen des zweiten Katheters zuführbar ist.

9. Katheter-System nach Anspruch 8, bei dem die therapeutische oder diagnostische Substanz aus der Gruppe von Medikamenten, Drogen, therapeutischen Agenzien, diagnostischen Agenzien. Myozyten, Myoblasten, anderen Zellen, genetischem Material, Gentherapie-Zubereitungen, Proteinen, Farbstoffen und strahlungsundurchlässigen Materialien ausgewählt ist.

10. Katheter-System nach einem der vorhergehenden Ansprüche, bei dem die optische Kohärenztomographie-Vorrichtung betreibbar ist, um zwischen einem Zielort und umgebenden anatomischen Strukturen oder Gewebe auf der Grundlage des Vorhandenseins oder des Fehlens eines metabolischen, physiologischen, genetischen und/oder Entwicklungs-Faktors zu unterscheiden.

11. Katheter-System nach einem der vorhergehenden Ansprüche, bei dem das Penetrator-Element eine Hohlnadel umfasst.

12. Katheter-System nach einem der vorhergehenden Ansprüche, bei dem die optische Kohärenztomographie-Vorrichtung betreibbar ist, um den Zielort durch Messen eines Charakterisierungs-Parameters zu lokalisieren, der aus der Gruppe von: metabolische Rate, pH, Gasgehalt, Temperatur, Bewegung, Zusammenziehbarkeit, Ischämie. Flüssigkeitsgehalt, Perfusion, Vorhandensein einer Infarzierung; Nekrose, Lebensfähigkeit und Gehalt an einer bestimmten Substanz ausgewählt ist.

13. Katheter-System nach einem der vorhergehenden Ansprüche, bei dem die optische Kohärenztomographie-Vorrichtung verwendbar ist, um die Stelle eines Zielortes zu bestimmen, der aus der Gruppe von: verletzbarer Plaque, infarziertem Gewebe, nekrotischem Gewebe, lebensfähigem Gewebe, spezifischen Neuronen innerhalb des Zentral-Nervensystems, spezifischen Nuklei innerhalb des Zentral-Nervensystems, ischämischem Gewebe, nicht-ischämischem Gewebe, Tumoren und neoplastischen Läsionen ausgewählt ist.

## Revendications

1. Système de cathéter de pénétration comprenant :
un cathéter allongé qui peut être inséré dans une lumière naturelle ou créée par l'homme dans le corps d'un patient mammifère;
un dispositif de pénétration (200) ayant une lumière de pénétration, ledit dispositif de pénétration pouvant être avancé du cathéter jusqu'à un emplacement cible à l'extérieur de la lumière, dans laquelle le cathéter est positionné ; et
un second cathéter (210) ayant une extrémité distale et une lumière, ledit second cathéter pouvant être avancé à travers la lumière du dispositif de pénétration (200) ;
**caractérisé en ce que** le système de cathéter de pénétration comprend en outre un appareil de tomographie à cohérence optique (100, 100a) positionné à l'intérieur de ou pouvant être inséré dans la lumière du second cathéter (210) et utilisable pour guider l'avancement du second cathéter (210) jusqu'à l'emplacement cible souhaité.

2. Système de cathéter selon la revendication 1, dans lequel l'appareil de tomographie à cohérence optique comprend un fil de tomographie à cohérence optique qui peut être inséré dans la lumière du second cathéter.

3. Système de cathéter selon la revendication 1, dans lequel l'appareil de tomographie à cohérence optique comprend une âme de tomographie à cohérence optique qui peut être insérée dans la lumière du second cathéter.

4. Système de cathéter selon l'une quelconque des revendications précédentes, dans lequel le système de cathéter comprend en outre au moins un marqueur (212) formé ou monté sur le second cathéter pour fournir une image ou artéfact sur l'image obtenue à partir de l'appareil de tomographie à cohérence optique (100, 100a).

5. Système de cathéter selon la revendication 4, dans lequel ledit au moins un marqueur (212) comprend un marqueur qui indique la position de l'extrémité distale du second cathéter.

6. Système de cathéter selon la revendication 5, dans lequel ledit au moins un marqueur comprend une bande de marqueur positionnée de manière adjacente à l'extrémité distale du second cathéter.

7. Système de cathéter selon l'une quelconque des revendications précédentes, en combinaison en outre avec un appareil pour délivrer une substance thérapeutique ou de diagnostic par le second cathéter.

8. Système de cathéter selon la revendication 7, en combinaison en outre avec une quantité d'une substance thérapeutique ou de diagnostic pouvant être délivrée par la lumière du second cathéter.

9. Système de cathéter selon la revendication 8, dans lequel la substance thérapeutique ou de diagnostic est choisie dans le groupe comprenant : des médicaments, des drogues, des agents thérapeutiques, des agents de diagnostic, des myocytes, des myoblastes, d'autres cellules, un matériel génétique, des préparations de thérapie génique, des protéines, des colorants et des matériaux radio-opaques.

10. Système de cathéter selon l'une quelconque des revendications précédentes, dans lequel l'appareil de tomographie à cohérence optique fonctionne pour distinguer l'emplacement cible des structures anatomiques ou tissus périphériques basé sur la présence ou l'absence d'un facteur métabolique, physiologique, génétique et/ou de développement.

11. Système de cathéter selon l'une quelconque des revendications précédentes, dans lequel le dispositif de pénétration comprend une aiguille creuse.

12. Système de cathéter selon l'une quelconque des revendications précédentes, dans lequel l'appareil de tomographie à cohérence optique fonctionne pour localiser l'emplacement cible en détectant un paramètre de caractérisation choisi dans le groupe comprenant : un taux métabolique, le pH, la teneur en gaz, la température, le mouvement, la contractilité, l'ischémie, la teneur en fluide, la perfusion, la présence d'infarctus ; la nécrose, la viabilité et la teneur en une substance particulière.

13. Système de cathéter selon l'une quelconque des revendications précédentes, dans lequel l'appareil de tomographie à cohérence optique peut être utilisé pour déterminer l'emplacement d'un emplacement cible choisi dans le groupe comprenant : une plaque vulnérable, un tissu infarci, un tissu nécrotique, un tissu viable, des neurones spécifiques dans le système nerveux central, des noyaux spécifiques dans le système nerveux central, un tissu ischémique, un tissu non ischémique, des tumeurs et des lésions néoplasiques.
